# EUROPÄISCHE PATENTSCHRIFT

(11) **EP 4 124 240 B1**
(45) Veröffentlichungstag und Bekanntmachung des Hinweises auf die Patenterteilung: **11.09.2024**
(21) Anmeldenummer: 22187055.3
(22) Anmeldetag: 26.07.2022
(51) Int. Cl.: A01K 1/03, A01H 4/00, B25J 21/02

(54) **REINRAUM-ZUCHTEINRICHTUNG ODER -WECHSELSTATION**
CLEAN ROOM BREEDING OR CULTIVATION DEVICE
DISPOSITIF DE CULTURE OU D'ÉLEVAGE EN SALLE BLANCHE

(30) Priorität: 27.07.2021 DE 102021119482
(43) Veröffentlichungstag der Anmeldung: 01.02.2023
(73) Patentinhaber: Hammelbacher, Stephan, 83684 Tegernsee (DE)
(72) Erfinder: Hammelbacher, Stephan, 83684 Tegernsee (DE); Fuchs, Alexander, 5030 Salzburg (AT)
(74) Vertreter: Beckord & Niedlich Patentanwälte PartG mbB

(56) Entgegenhaltungen:
- WO-A1-2018/007596
- US-A1- 2007 079 765
- US-A1- 2017 339 910

## Beschreibung

Die Erfindung betrifft eine karussellartige Zuchteinrichtung bzw. eine Wechselstation für die Tier- oder Pflanzenforschung. Sie ist als Reinraumeinrichtung zum Herstellen und zum Aufrechterhalten einer keimfreien Umgebung und damit als Isolator ausgebildet. Sie ist mit einem drehbaren zylindrischen Regal mit einer Hochachse als Drehachse, mit scheibenförmigen Tellern mit Stellplätzen zum Abstellen von Behältern, mit einer Hubeinrichtung- und mit einer Dreheinrichtung für die scheibenförmigen Teller, mit im Regal ringförmig anzuordnenden und in radialer Richtung entnehmbaren, im Wesentlichen sektorförmigen Behältern, mit einer Hülle zur sterilen Abschirmung des Regals gegenüber der Umgebung und mit einer Schnittstelle an der Hülle zum Anschluss eines Handschuhkastens (engl.: Glovebox) ausgestattet. Die Wechselstation verfügt außerdem regelmäßig über eine Arbeitsplatte innerhalb der Hülle, um dort mindestens zwei Behälter gleichzeitig abstellen und sie oder ihren Inhalt manipulieren zu können. Die Hülle bietet eine sterile Abschirmung des Regals gegenüber der Umgebung, sodass es sich bei der Zuchteinrichtung bzw. bei der Wechselstation um einen Isolator handelt.

In der präklinischen Forschung mit Tieren und Pflanzen verhindern externe Einflüsse, wie Viren, Bakterien, Chemikalien, Klima und unterschiedliche Versuchsumgebungen die globale Reproduzierbarkeit von Ergebnissen.

Eine ähnliche Problematik stellt sich der Pharmaindustrie sowohl in der Forschung mit Tieren und Pflanzen, als auch bei der Gewinnung von Wirkstoffen mit Hilfe von Pflanzen und deren Vermehrung dar. Pflanzen (Glycaneering) stellen z. T. effizientere Bioreaktoren als Tiere dar. Auch in diesem Bereich sind absolut kontrollierte Bedingungen für Zucht und Experiment erforderlich.

Die WO 2018 007 596 A1 zeigt einen gattungsgemäßen Karussellisolator. Er erfüllt neben den zuchttechnischen Bedingungen außerdem auch ergonomische Anforderungen. Seine Konstruktion ist auf einen Einsatz vor allem im Tierzuchtbereich hin optimiert. Seine Merkmale bilden die Basis der Erfindung.

Aufgabe der Erfindung ist es, eine Zuchteinrichtung bzw. eine Wechselstation zur Verfügung zu stellen, die ein breiteres Anwendungsspektrum bietet.

Diese Aufgabe wird bei der eingangs genannten Wechselstation erfindungsgemäß durch eine Wasserversorgungseinrichtung für die Behälter gelöst, die in der Wechselstation selbst angeordnet ist und die eine Versorgung jedes einzelnen Behälters und/oder aller Behälter zusammen ermöglicht. Weil sie vollständig innerhalb der Hülle der Wechselstation untergebracht ist, arbeitet die Wasserversorgungseinrichtung sogar unter den strengen Bedingungen eines Isolators. Damit gelingt es, eine automatisierte und miniaturisierte Haltungs-, Zucht- und Experimentplattform für Nager, Fische, Pflanzen, Mikroorganismen etc. zu bieten, die modular konfiguriert werden kann. Sie kann alle Funktionen zur Durchführung und Dokumentation von Tier- und Pflanzenversuchen bzw. deren Aufzucht und Vermehrung, ggf. sogar in einer zu 100% kontrollierten und keimfreien Umgebung bzw. einen Reinraum bieten, ggf. über den gesamten Lebenszyklus hinweg und bis hin zu Ernte und Verkauf. Denn insbesondere Tierversuche werden trotz ihrer Notwendigkeit von einem breiten Teil der Bevölkerung abgelehnt. Es ist langfristig davon auszugehen, dass keine großen Versuchstiereinrichtungen mehr gebaut werden, sondern kleinere und skalierbarere Lösungen gesucht werden.

Die Erfindung ermöglicht es, identische und global verteilte Datenerfassungsumgebungen für präklinische Experimente mit Tieren oder mit Pflanzen mit zentralen Auswertungsmöglichkeiten zu bieten. Global verteilte, datentechnisch vernetz- und synchronisierbare Reinraumeinheiten ermöglichen eine einheitliche Datenerfassung. Durch die Vernetzung von beispielsweise auch klima- und lichttechnisch synchronisierten Einheiten kann überregional an einem Projekt unter den gleichen Bedingungen gearbeitet werden. Die Speicherung der so erfassten Daten in einer zentralen Datenbank schafft neue Möglichkeiten der Auswertung.

Darüber hinaus lässt sich das erfindungsgemäße System auch im aufstrebenden Markt des "Vertikal Farming" einsetzen, sogar bis hin zu Systemen mit Kundenkontakt, indem Endverbrauchermärkte ihren Kunden z.B. frische Kräuterpflanzen ohne Einsatz von Pestiziden und Kunstdüngern und ohne lange Transportwege anbieten können.

Die erfindungsgemäße Wechselstation kann als modulares System aufgebaut sein, bei dem Teller mit zentralen Hohlräumen und mit Stellplätzen zum Abstellen von Behältern übereinander angeordnet, zum Beispiel aufeinander gesteckt werden, um unterschiedlich geeignete Bauhöhen erreichen zu können. Die aus den zentralen Hohlräumen der Teller entstehende Röhre kann für die Wasserversorgungseinrichtung mit mindestens jeweils einer Kopplungsstelle an jedem Stellplatz, für Zu- oder Abluft der Container und/oder für elektrische Verkabelungen verwendet werden. Als Antrieb für den vorzugsweise teleskopierenden Hub- und Drehmechanismus können in einem Balg im Sockel Elektromotoren untergebracht sein. Je nach Bauhöhe und Lage der Arbeitsplatte können Benutzer auf dem Boden, auf einem Podest oder einem Gerüst stehen, um genügend Bodenfreiheit für den Hub und die Senkung des Regals zu bekommen. So lassen sich auch hohe Regale für die massenhafte Vermehrung auf kleinster Grundfläche umsetzen.

Für die Steuerung und die Dokumentation aller Transaktionen mit den Tieren oder Pflanzen bzw. deren Behältern existiert bereits ein Betriebssystem für Wechselstationen (CSOS), ein Verfahren, welches die fachlichen Prozesse von Vorratshaltung, Zucht und Experiment mit den erforderlichen Bewegungen, Leistungen und Befunden der Objekte des Interesses, also der Tiere, der Pflanzen etc. und deren Unterbringungsort und den Besitzer (Screen) im Zusammenspiel mit einer transpondergestützten bzw. durch optische Codes gestützten Wechselstation abbildet (s. US 9 349 031 B2). Das Betriebssystem kann um die Antriebssteuerung und die Klima- und Wassersteuerung mit der Verwaltung der dazugehörigen Sensorik und ihrer Parameter erweitert werden.

Damit steht eine Plattformlösung zur Verfügung, die Forschungsobjekte und deren Veränderungen bzw. Befunde, wie auch die Umgebung, in der sie zustande kommen, ganzheitlich verwaltet. Sie bietet neue Ansätze für die Forschung, eine deutliche Verbesserung des Tierwohls, der Effizienz und der Arbeitsumgebung für die Benutzer.

Erfindungsgemäß ist die Wasserversorgungseinrichtung für die Behälter in der Wechselstation selbst untergebracht. Nach einer vorteilhaften Ausgestaltung der Erfindung kann sie auf zumindest einem Teller des Regals angeordnet sein. Sie kann dadurch unabhängig von einer Aktivität der Hubeinrichtung und/oder der Dreheinrichtung der Wechselstation arbeiten. Sie stellt insofern eine autarke, nämlich ggf. mitgedrehte und mitgehobene Wasserversorgung der Behälter sicher.

Die Wasserversorgungseinrichtung umfasst vorzugsweise zumindest einen Wasservorratstank und eine Wasserpumpe zur Versorgung der Behälter. In der Tierforschung bekannt sind in Reihe bzw. seriell angeordnete Wasserzapfstellen für jeden Tierkäfig. Die erfindungsgemäße Wasserversorgungseinrichtung umfasst dagegen einen Vorlauf vom Wasservorratstank zu jedem Behälter und einen davon getrennten Rücklauf von jedem Behälter zurück zum Wasservorratstank. Der vom Rücklauf getrennte Vorlauf jedes Behälters ermöglicht damit nicht nur eine Wasserversorgung jedes Behälters, sondern für jeden Behälter auch eine Wasserentsorgung. Damit eignet sich die Wechselstation für den Einsatz jedenfalls auch in der Pflanzenzucht.

Erfindungsgemäß kann jedem Teller der Wechselstation jeweils eine ringförmige Leitung als Vorlauf und eine ringförmige Leitung als Rücklauf zugeordnet sein. Die ringförmigen Leitungen können im Regal koaxial mit dessen Drehachse angeordnet sein. Damit lässt sich eine platzsparende Wasserversorgung der Behälter in der Wechselstation sicherstellen.

Die ringförmigen Leitungen können jeweils Kopplungsstellen zum Koppeln mit den Behältern bieten. Demzufolge kann die Wechselstation bzw. ihr Regal jeweils an jeden Stellplatz eine erste Kopplungsstelle für einen Wasserzufluss zu einem Behälter als Vorlauf und jeweils eine zweite Kopplungsstelle für den Wasserabfluss von demselben Behälter als Rücklauf umfassen. Die Wechselstation kann darüber hinaus entsprechende Behälter mit jeweils zwei mit den obigen Kopplungsstellen zusammenwirkende Gegenkopplungsstellen aufweisen. Jedem Stellplatz zum Abstellen eines Behälters in der Wechselstation kann folglich ein Paar aus einer ersten und einer zweiten Kopplungsstelle zugeordnet sein. Jeder Behälter kann seinerseits über ein Paar an entsprechenden Gegenkopplungsstellen verfügen. Die Gegenkopplungsstellen koppeln beim korrekten Abstellen des Behälters auf seinem Stellplatz in der Wechselstation mit dem Kopplungsstellen des Regals.

Der Wasservorratstank der Versorgungseinrichtung kann als Zwischen- oder als Pufferspeicher dienen, der von außen zugeführtes Wasser aufnimmt. Er kann einen Vorrat bilden, um Wassermenge zu puffern oder um eine Zubereitung für die weitere Verwendung zu ermöglichen. Nach einer weiteren vorteilhaften Ausgestaltung der Erfindung kann die Wechselstation einen geschlossenen Wasserkreislauf der Wasserversorgungseinrichtung aufweisen. Der geschlossene Wasserkreislauf kann die Wechselstation unabhängig von einer Wasserzufuhr und damit unabhängig von einer entsprechenden Installation machen. Dadurch kann der Standort der Wechselstation unabhängig von Wasserinstallationen gewählt werden, was die Einsatzmöglichkeiten der Wechselstation erweitert.

Je nach der zur Verfügung stehenden Qualität des Wassers für die Wasserversorgungseinrichtung kann es erforderlich sein, das Wasser vor dem Einsatz in der Wechselstation aufzubereiten. Nach einer weiteren vorteilhaften Ausgestaltung der Erfindung kann die Wechselstation daher eine Reinigungseinrichtung für das Wasser der Wasserversorgungseinrichtung umfassen. Sie kann nicht nur bei einer externen Wasserversorgung notwendig sein, sondern auch bei einem geschlossenen Wasserkreislauf, wenn das Wasser des Rücklaufs sich nicht unbehandelt als Wasser für den Vorlauf eignet. Einen geschlossenen Wasserkreislauf kann die Reinigungseinrichtung so u.U. erst ermöglichen, wenn sie das Wasser des Rücklaufs reinigt, um es als Wasser des Vorlaufs verwendbar zu machen.

Nach einer weiteren vorteilhaften Ausgestaltung der Erfindung kann die Wechselstation zweckmäßiger Weise eine Dosiereinrichtung für das Wasser der Wasserversorgungseinrichtung aufweisen. Sie kann einen geeigneten Zufluss zu den Behältern und / oder einen sparsamen Wassereinsatz sicherstellen.

Nach einer weiteren vorteilhaften Ausgestaltung der Erfindung kann die Wechselstation eine Belüftungs- und/oder Klimatisierungseinrichtung aufweisen. Sie erlauben das Einstellen einer notwendigen oder gewünschten Atmosphäre weitgehend unabhängig von gewählten Aufstellort der Wechselstation. Auch dadurch können sich die Einsatzmöglichkeiten der Wechselstation erweitern lassen, weil der Standort der Wechselstation weitgehend unabhängig von klimatischen Verhältnissen gewählt werden kann. Die erfindungsgemäße Wechselstation ermöglicht damit den Aufbau global verteilter und klima-, wasser- und lichttechnisch synchronisierter und ggf. datentechnisch vernetzter und synchronisierter Wechselstationen oder Reinraumeinheiten. Damit kann unter den gleichen Bedingungen überregional an demselben Projekt gearbeitet werden.

Jede Forschungsaufgabe kann eigene Voraussetzungen erfordern, wobei sich diejenigen der Tierforschung von denjenigen der Pflanzenforschung regelmäßig unterscheiden. Sie betreffen insbesondere die Behälterausstattung. Nach einer weiteren vorteilhaften Ausgestaltung der Erfindung kann die Wechselstation daher einen Behälter mit einer schiefen Ebene darin und mit Abstellplätzen für Pflanzen auf der schiefen Ebene umfassen. Zur Versorgung der Pflanzen mit Wasser kann der Behälter eine erste, obere Gegenkopplungsstelle für den Wasserzufluss an einer Oberseite und eine zweite, untere Gegenkopplungsstelle für den Wasserabfluss an einer Unterseite der schiefen Ebene aufweisen. Die erste Gegenkopplungsstelle des Behälters kann sich an die erste Kopplungsstelle des Regals ankoppeln lassen, die mit dem Vorlauf bzw. mit dessen ringförmiger Leitung in Verbindung steht, die zweite Gegenkopplungsstelle ist folglich in gleicher Weise der zweiten Kopplungsstelle und damit dem Rücklauf zugeordnet. Der Vorlauf der Wasserversorgungseinrichtung kann dadurch Wasser regelmäßig oder schwallweise an die Wurzeln der auf der Oberseite der schiefen Ebene abgestellten Pflanzen fördern. Überschüssiges Wasser fließt von der geneigten Oberseite der schiefen Ebene ab und gelangt über die unterseitige zweite Gegenkopplungsstelle in den Rücklauf. Der erfindungsgemäße Behälter kann sich so für die Anwendung des NFT-Verfahrens (Nutrient-Film-Technik-Verfahren) eignen. Das NFT-Verfahren erlaubt eine gleichmäßige Versorgung der Pflanzenwurzeln mit sauerstoffreicher Nährlösung bei geringstem Wasserverbrauch in einem geschlossenen Wasserkreislauf.

Alternativ kann der Behälter zur Versorgung der Pflanzenwurzeln nach dem Ebbe-/Flutverfahren ausgelegt sein. Dazu kann er eine Zuflusskammer für Frischwasser bzw. frische Nährlösung umfassen, in den die Wurzeln der Pflanzen oder das Substrat der Samen hineinreichen. Darunter - unmittelbar oder versetzt, jedenfalls auf einer tieferen Ebene des Behälters - befindet sich dann eine Abflusskammer, die unbenutztes Wasser bzw. unbenutzte Nährlösung aufnimmt, das/die ein Ablauf von der Zuflusskammer liefert. Der Ablauf kann ein steuerbares Ventil enthalten oder für einen langsamen Abfluss geeignet dimensioniert sein. Dadurch lässt sich in der Zuflusskammer eine Zeit lang einen hohen Flüssigkeitsstand aufrechterhalten und ein vollständiger Abfluss in die Abflusskammer gewährleisten.

In beiden Fällen können einzelne Ebenen ggf. auch mit unterschiedlichen Nährlösungen versorgt und deren elektrische Leitfähigkeit permanent kontrolliert werden, um den Pflanzen auf der jeweiligen Tellerebenen eine optimale Osmose zu erlauben. Darüber hinaus lässt sich auch ein aquaponisches System aufbauen, bei dem die Nährlösung durch Fische im Rahmen eines Nitrationsprozesses von der Ausscheidung von Ammoniak zu Nitrat (NO₃) umgewandelt wird. Allgemein sorgen hydroponische Aufzuchtverfahren für einen günstigen ökologischen Fußabdruck bei maximaler Ausbeute.

Die Tierforschung dagegen stellt andere Anforderungen an eine Wechselstation. Nach einer alternativen Ausgestaltung der Erfindung kann die Wechselstation daher Behälter mit einer ersten Gegenkopplungsstelle für den Wasserzufluss und mit einer zweiten Gegenkopplungsstelle für den Wasserabfluss umfassen, die mit einer U-förmigen Leitung verbunden sind, die ein in die Richtung des Behälterinnenraums weisendes und von dort aus erreichbares Trinkventil für Tiere aufweist. Die relative Lage der Gegenkopplungsstellen des Behälters für die Tierforschung ist dafür weitgehend beliebig, solange sie sich über einen U-förmigen Leitungsabschnitt miteinander verbinden lassen. Auch Behälter für die Tierforschung mit zwei Gegenkopplungsstellen auszustatten bietet den Vorteil, dass sich ihr Aufbau insofern nicht von demjenigen für die Pflanzenforschung unterscheidet.

Die Regale weisen folglich keinen Unterschied auf, der im unterschiedlichen Einsatzzweck für die Pflanzen- oder für die Tierforschung begründet wäre, was Kostenvorteile bietet. Auch die Herstellung der Behälter kann somit kostengünstiger sein, weil sie sich trotz unterschiedlichen Einsatzzwecks im Aufbau überwiegend gleichen.

Weil jede Anwendung andere Voraussetzungen erfordert, sich also beispielsweise diejenigen der Tierforschung von denjenigen der Pflanzenforschung deutlich unterscheiden, verfolgt die Erfindung das Prinzip, die übereinstimmenden Anforderungen im oder mit dem Regal der Wechselstation zu erfüllen und die Unterschiede möglichst in den Aufbau der Behälter zu verlegen. Das Regal schließt dabei seine Hülle, seine Arbeitsfläche, die Wasserversorgungseinrichtung und seine Antriebe ein und kann ggf. zum Beispiel um Belichtungs-, Belüftungs-, Klimatisierungs- und Dosierungstechnik, Handschuhkasten, Schleusen, zum Beispiel auch mit DPTE^{®} (Double Porte pour Transfert Etanche), einen Cobot, CageTalkers^{®} etc. ergänzt werden. Die erfindungsgemäße Wechselstation kann also als Basisplattform konzeptioniert sein, die in beiden Bereichen gleichermaßen eingesetzt werden kann.

Eine weitere Optimierung liegt darin, auch die Behälter an sich identisch aufzubauen, die Unterschiede also in die Ausstattung des Behälterinnenraums jenseits der Gegenkopplungsstellen zu verlagern. Nach einer weiteren vorteilhaften Ausgestaltung der Erfindung kann die Wechselstation daher Behälter umfassen, deren schiefe Ebene als Teil eines austauschbaren Einsatzes zum Erzeugen eines Behälters für die Pflanzenforschung ausgebildet ist. Der entnehmbare Einsatz mit der schiefen Ebene kann so konstruiert sein, dass er im Behälter eingesetzt die erste Gegenkopplungsstelle auf einer Oberseite der schiefen Ebene und die zweite Gegenkopplungsstelle auf deren Unterseite platziert.

Die Wechselstation kann außerdem Behälter umfassen, die einen U-förmigen Leitungsabschnitt zum fluidischen Verbinden der Gegenkopplungsstellen als Teil eines austauschbaren Einsatzes umfasst. Der U-förmige Leitungsabschnitt verbindet nicht nur die Gegenkopplungsstellen des Behälters miteinander, sondern ragt außerdem in den Behälterinnenraum hinein. Dort bietet er mit einem Trinkventil für Tiere einen Zugang zu Trinkwasser, so dass der Einsatz einen Behälter für die Tierforschung ausstatten kann.

Die erfindungsgemäße Wechselstation stellt also ein System aus einem Regal und den zugehörigen Behältern dar, die für alle Anwendungen identisch aufgebaut sind. Außerdem umfasst das erfindungsgemäße System eine der Behälteranzahl entsprechende Anzahl an Einsätzen, die sich je nach Anwendung in der Tiefforschung oder in der Pflanzenforschung voneinander unterscheiden. Das System bietet damit eine hohe Anzahl an Gleichteilen bzw. eine sehr geringe Anzahl an unterschiedlichen Bestandteilen für die unterschiedlichen Einsatzzwecke. Denn je nach Anwendungsgebiet müssen nur die jeweiligen Einsätze individuell ausgewählt werden. Die weit überwiegende Anzahl an Gleichteilen ermöglicht eine kostengünstige Herstellung des Regals und der Behälter.

Ein Behälter für die Pflanzenforschung lässt sich durch die Entnahme seines Einsatzes mit der schiefen Ebene und durch Austausch gegen einen Einsatz mit einem U-förmigen Leitungsabschnitt zum fluidischen Verbinden der Gegenkopplungsstellen sogar zu einem Behälter für die Tierforschung umrüsten und umgekehrt.

Nach einer weiteren vorteilhaften Ausgestaltung der Erfindung kann die Wechselstation daher Behälter umfassen, die einen Durchbruch einer Seitenwand zum Umsetzten von Tieren aufweisen, wobei der Durchbruch derart angeordnet ist, dass die Durchbrüche zweier Behälter bei einem benachbarten bzw. bei einem Nebeneinander-Positionieren der Behälter Seite an Seite miteinander zur Deckung kommen. Auch bekannte Behälter haben Durchbrüche in einer Seitenwand. Um Behälter auf ihren Regalplätzen tauschen zu können, liegen die Durchbrüche bekannter Behälter an derselben Position. Erfindungsgemäß sind die Durchbrüche jedoch außerdem derart gestaltet, dass sie bei einem Nebeneinander-Positionieren der Behälter Seite an Seite nicht nur geometrisch miteinander fluchten, sondern derart mit miteinander zur Deckung kommen, dass sie eine spaltfreie Passage von dem einen Behälter zum anderen bilden. Sie kommen also derart zur Deckung, dass sie sich bzw. ihre offene Durchbruchsfläche gegenseitig vollständig bedecken und die Ränder der Durchbrüche über den gesamten Umfang der Durchbrüche hinweg unmittelbar, nämlich ohne Zwischenschaltung weiterer Bauteile, aneinander anliegen.

Die Anordnung verfolgt das Prinzip, die Tiere ihren Ortswechsel selbst und aus eigener Kraft vornehmen zu lassen. Mangels menschlichen Eingriffs werden die Tiere weniger beunruhigt und nicht unter Stress gesetzt, was dem Tierwohl zugutekommt. Damit wird insbesondere das Risiko eines Stresskollapses bei sensiblen Tierstämmen aufgrund einer Beengung durch Zugriff mit der Hand aufgrund des Fluchttriebes der Tiere komplett ausgeschlossen. Darüber hinaus lässt sich erfindungsgemäß nicht nur ihre Bewegungsfähigkeit und Bewegungsfreude, sondern auch die Motivation der Tiere zum Ortswechsel nutzbar machen, weil insbesondere Mäuse neugierig sind und eine angebotene neue Passage von sich aus erkunden. So müssen die Tiere nicht einmal mehr mobilisiert werden, um die Passage von ihrem bisherigen in den neuen Behälter zu nehmen. Selbst verhältnismäßig stressarme Umsetzmethoden zum Beispiel mit Handlingsröhrchen lassen sich erfindungsgemäß also bequem, sicher und vollständig ersetzen.

Vorteilhaft sind die Durchbrüche der Behälter also deckungsgleich. Um spaltfrei aneinander anliegen zu können, können sie bezüglich einer Außenfläche derjenigen Seitenwand des Behälters, in der sie liegen, plan angeordnet sein oder behälteraußenseitig überstehen. Die sie umgebenden Ränder jedenfalls sind weitgehend eben, so dass die spaltfrei aneinander anliegenden, sich deckenden Durchbrüche insoweit zwar nicht zwingend dichtend, vor allem nicht gas- oder wasserdicht sein müssen, aber jedenfalls den Tieren keine Veranlassung geben, an der Kontaktstelle der nebeneinanderliegenden Durchbrüche Fluchtmöglichkeiten zu wittern.

Die Form und die Abmessungen der Durchbrüche können sich, von der Größe der Tiere abgesehen, zum Beispiel an praktischen, wie herstellungsseitigen oder pflegeseitigen Aspekten orientieren. Nach einer vorteilhaften Ausgestaltung der Erfindung sind die Abmessungen des Durchbruchs, beispielsweise der Durchmesser eines kreisförmigen Durchbruchs, auf die Abmessungen der Tiere derart angepasst, dass jeweils nur genau ein Tier einen Durchbruch passieren kann. Damit kann während des Umsetzens eine Vereinzelung der Tiere vorgenommen werden, was beispielsweise ein Zählen der Tiere erleichtert oder eine Auswahl von Individuen ermöglicht.

Damit die Tiere nicht ungewollt den Behälter durch den Durchbruch hindurch verlassen können, verfügt er über einen Verschluss. Nach einer weiteren vorteilhaften Ausgestaltung kann der Behälter über einen in der Erstreckungsebene der durchbrochenen Seitenwand des Behälters verlagerbaren Verschluss des Durchbruchs verfügen. Er kann verschiebbar oder um eine Achse verschwenkbar sein, die auf die Erstreckungsebene der durchbrochenen Seitenwand senkrecht verläuft. Er kann manuell oder maschinell oder beides bedienbar sein. Seine Verlagerbarkeit vermeidet ein ungewolltes Öffnen, sei es durch die Tiere von innen oder durch unbedachtes Hantieren von außen. Die Betätigungskraft des verlagerbaren Verschlusses lässt sich zudem mit einfachen Mitteln anpassen oder ändern. Außerdem bietet die Wahl der Verlagerungsrichtungen des Verschlusses große Freiheiten. Denn die Betätigungsrichtung kann in der Erstreckungsebene der durchbrochenen Seitenwand des Behälters nahezu beliebig gewählt werden, womit auf die Art der Bedienung des Verschlusses - zum Beispiel manuell oder maschinell - oder auf Platzanforderungen flexibler reagiert werden kann.

Der Durchbruch kann im oben genannten Rahmen grundsätzlich in jeder beliebigen Seitenwand eines Behälters angeordnet sein. Bei verschiedenen Behältern können die Durchbrüche ggf. sogar in unterschiedlichen Seitenwänden angeordnet werden, solange sich die Behälter nebeneinander positionieren und sich die Durchbrüche dabei zur Deckung bringen lassen. Vorteilhaft befinden sich die Durchbrüche mehrerer Behälter in derselben Seitenwand und an derselben Stelle des Behälters. Außerdem kann der Verschluss der Durchbrüche jeweils ein luftdurchgängiges Gitter oder Filter umfassen. Das Gitter oder Filter kann dazu am Verschluss angebracht sein oder selbst den Verschluss bilden. Damit kann der Verschluss eine Doppelfunktion erfüllen, nämlich neben der Erleichterung des Umsetzens eine Luftzu- oder -abfuhr ermöglichen. Um diese Funktion zu erfüllen, ist der Durchbruch zweckmäßig an allen Behältern einer Zuchteinrichtung an derselben Stelle angeordnet. Dafür eignet sich insbesondere eine der Drehachse des Regals zugewandten Schmalseite des Behälters. Der Luftein-/-auslass kann damit auch als Durchbruch zum Umsetzten von Tieren dienen.

Insbesondere, wenn ein Durchbruch zum Umsetzen der Tiere nicht auf einer Schmalseite des Behälters angeordnet ist, können schildförmige Leitplatten beim Umsetzen helfen. Sie können in Behälterecken beidseits der Durchbrüche einsetzbar oder eingesetzt sein und die die Behälterecken abschirmen. Sie geben dem Behälter durchbruchseitig eine Art Trichterform, die eine Mobilisierung der Tiere auf den Durchbruch hin erleichtert. Dauerhaft abgeschirmte Ecken des Behälters können zur Aufnahme von Utensilien in Griffweite des Bedieners der Zuchteinrichtung genutzt werden.

Vor allem die erfindungsgemäße Wechselstation, insbesondere aber die erfindungsgemäßen Behälter für die Tierzucht ermöglichen also ein Verfahren zum Betreiben einer Wechselstation und zwar insbesondere zum Umsetzen von Tieren mit den folgenden Schritten:
a) Entnahme zweier Behälter aus dem Regal, die jeweils mit den oben beschriebenen Durchbrüchen in einem ihrer Behälterwände versehen sind,
b) Positionieren der mit einem Durchbruch versehenen Behälterwände nebeneinander, so dass die Durchbrüche sich decken,
c) Öffnen der Durchbrüche,
d) Mobilisieren eines oder mehrerer Tiere aus dem einen Behälter in Richtung auf den Durchbruch hin, sofern die Tiere nicht von sich aus wandern,
e) Verschließen der Durchbrüche,
f) Zurückstellen der Behälter in das Regal, sofern sich nicht andere Maßnahmen, als Vorgänge oder Leistungen an einem oder an den beiden Behältern anschließen sollen.

Das Verfahren ist also noch schonender den Tieren gegenüber, weil ein Käfigwechsel im Regelfall ganz ohne Eingriff von außen erfolgen kann. Einer der entnommenen Behälter kann Beispiel mit Tieren besetzt sein, der andere leer. Die Entnahme des besetzten Behälters aus dem Regal stellt für die Tiere keinen unbekannten Vorgang dar. Durch Positionieren des leeren Behälters mit seinem Durchbruch deckungsgleich mit und dem Durchbruch des besetzten Behälters und durch Öffnen der Durchbrüche bietet sich den Tieren eine neue Passage, die sie in der Regel erkunden wollen. Bei geeigneter Abmessung der Durchbrüche können die Tiere sie nur einzeln passieren, so dass ein gezieltes Wechseln der Tiere vom besetzten in den zunächst leeren Behälter möglich ist. Ist der Zielzustand z. B. nach Anzahl der Tiere im anfangs leeren Behälter erreicht, lassen sich die Durchbrüche verschließen. Sofern keine weiteren Maßnahmen, wie etwa Ausstatten des ursprünglich leeren Behälters mit Futter, Untersuchung der Tiere oder dergleichen vorgesehen ist, können die Behälter in das Regal zurückgestellt werden. Für die Durchführung des Umsetzverfahrens kommt es also insbesondere auf die oben beschriebene Ausgestaltung der Behälter mit Durchbrüchen an. Eine Ausstattung der Wechselstation mit einer oben beschriebenen zentralen Wasser- oder Luftversorgung ist an sich nicht zwingend erforderlich, aber im Interesse der Qualität der Tierhaltung wünschenswert.

Der begrenzte Raum in der Wechselstation erfordert eine genaue Planung der Zucht von Tieren, so dass auch das Risiko der Produktion von überschüssigen Tieren begrenzt wird. Das erfinderische Konzept bietet darüber hinaus die Möglichkeit, innerhalb der Hülle einen Cobot für die Versorgung der Tiere einzusetzen und damit Personal einzusparen bzw. das Risiko der Vernachlässigung aufgrund von Personalmangel zu begrenzen. Derartige Vernachlässigungen wurden von Presse wiederholt aufgegriffen und angeprangert.

Aufgrund der kurzen Wege vom Regal zur Arbeitsplatte und dem erfinderischen Umsetzverfahren kann ohnehin schon von deutlich weniger Stress für die Tiere als bei traditionellen Verfahren ausgegangen werden. Da das obige Umsetzverfahren außerdem automatisiert werden kann, kann der Einsatz eines Roboters oder Cobots für das Umsetzen einen Stresskollaps der Tiere beim Umsetzen weitgehend ausschließen.

Als Isolator ermöglicht die erfindungsgemäße Wechselstation nicht nur die Forschung in der Gnotobiotik, mit dem Microbiom, und der "Altered Schaedler Flora" (ASF), also den klassischen Anwendungsfeldern von Isolatoren, sondern ist auch für alle andere Einsatzfelder in der Tierforschung und für eine räumlich unabhängige Zusammenarbeit an verschiedenen Isolatoren geeignet.

Die besonderen Vorteile der erfindungsgemäßen Isolatoren gegenüber traditionellen Tierhauseinrichtungen, wie offener Haltung, IVC-Haltung, Tierschrankhaltung oder traditioneller Isolatorenhaltung liegen in der garantierten Hygiene und dem besseren Schutz von Mensch und Tier, in der Skalierbarkeit der Kapazität, Flexibilität, Mobilität und Automatisierbarkeit. Dazu kommen die effizientere Nutzung des verfügbaren Platzes, größere Nachhaltigkeit beim Verbrauch von Wasser, Chemikalien und Verbrauchsmaterial. Je nach Hygieneanspruch, Anwendungszweck und Budget lassen sich die erfindungsgemä-ßen Isolatoren als flexible Film-Isolatoren oder "hardwalled" mit Schleusen für die unterschiedlichen Ansprüche an die Isolation im Handschuhkasten integrieren.

Die erfindungsgemäße Wechselstation kann aber auch ein effizientes Verfahren für die Zucht, Produktion und Vermehrung von Jungpflanzen auf kleinstem Raum darstellen. Für den Einsatz der Wechselstation im Bereich des Vertical Farming kann sie eine Ausgabeeinrichtung für Pflanzen, insbesondere mit einem Industrieroboter zur Manipulation der Behälter und ihres Inhalts innerhalb der Hülle und einem außenseitigen Entnahmegestell zur benutzerseitigen Entnahme und mit einem Anschluss an die Schnittstelle an der Hülle umfassen. Durch die Automatisierung der Wechselstation mit Hilfe des Dreh- und Hubmechanismus in Verbindung mit einem vertikal laufenden robotischen Pickmechanismus können vollständige Pflanzencontainer auf dem Entnahmegestell serviert werden, aus denen ein Benutzer dann einzelne Pflanzen entnehmen kann. Über eine Fotozelle kann beim Zurückstellen des Containers kontrolliert, ob die Anzahl der angeforderten Pflanzen den entnommenen Pflanzen entspricht.

Optional kann ein Ernte- bzw. Verkaufsregal vorab bestückt werden, indem mit Hilfe eines Cobots oder eines erweiterten Pickmechanismus zum Beispiel einzelne Pflanztöpfe automatisch dem Regal der Wechselstation entnommen und im Verkaufsregal platziert werden. Dann kann ein Benutzer bzw. Kunde die Pflanzen direkt aus dem Verkaufsregal entnehmen und registrieren, wie heute bereits in Supermärkten üblich. Personal für die Bonnierung und die Ernte kann so weitgehend eingespart werden.

Im Bereich der Pflanzenzucht und der Pflanzenforschung stellt die erfindungsgemäße Wechselstation ein gewisses Neuland dar. Hier kann sie der laufenden Forschung, Pflanzen widerstandsfähiger und ertragreicher zu machen, gute Dienste leisten. Denn die Möglichkeit, die Wechselstation auch hermetisch zu isolieren, dürfte neue, systematische Forschung mit Mikroben beflügeln.

Die erfindungsgemäße Wechselstation kann aufgrund ihrer weitgehend oder komplett geschlossenen Behälter sowohl in der Pflanzenforschung wie auch in der Tierforschung zu mehr Hygiene und Schutz von Mensch und Pflanze bzw. Tier und dadurch auch zu einem deutlich geringeren Verbrauch von Schutzkleidung führen. Sie bietet eine weitgehende Standardisierbarkeit und Reproduzierbarkeit der Umgebung, womit sie bessere Forschungsergebnissen ermöglicht. Ein geringerer Wasser- und Chemikalienverbrauch lässt sich durch die Wasserversorgung und die Dosiereinrichtung erreichen. Außerdem trägt sie zur Vermeidung von Engpässen an einer weiterhin erforderlichen Laminar-Flow-Station bei, macht sie zum Beispiel für die Wurzelbeschneidung von Setzlingen gar entbehrlich.

Die erfindungsgemäße Wechselstation ermöglicht eine gewisse Verkleinerung von Zuchteinheiten, die aber vollständig und automatisch versorgt werden können. Daher bietet sie die Möglichkeit, ihre Kapazität zu skalieren, steigert die Mobilität und Flexibilität der Forschungsumgebung, bietet kurze Wege, eine hohe Verfügbarkeit und eine gute Ausnutzung verfügbaren Platzes.

Die Automatisierung der erfindungsgemäßen Wechselstation erlaubt einfachere Handling-Möglichkeiten im Vergleich zu traditionellen Plantcubes oder Isolatoren, eine hohe Zeitersparnis bei der Wasserversorgung, eine effizientere Lagerung, eine Standardisierung der Arbeitsprozesse in der Wechselstation, eine Reduktion der menschlichen Interaktion, selbst bei Ernte und Verkauf in der Pflanzenzucht und der in der Tierzucht insbesondere Tierstress reduzieren kann, eine "Remote"-Bedienung und Beobachtung der Wechselstation und letztlich weniger Personal.

Das Prinzip der Erfindung wird im Folgenden anhand einer Zeichnung beispielshalber noch näher erläutert. In der Zeichnung zeigen:
- Figur 1:: eine erste Ausführungsform eines erfindungsgemäßen Karussell-Isolators mit Handschuhkasten und Abfallbehälter,
- Figur 2:: eine Schnittansicht des Karussell-Isolators der Figur 1,
- Figur 3:: eine zweite Ausführungsform des erfindungsgemäßen Karussell-Isolators mit einem Ausgabeschacht,
- Figur 4:: eine Teilansicht der Wasserversorgung des Isolators der Figuren 1 und 2,
- Figur 5:: eine Teilansicht der Wasserversorgung gemäß Figur 4,
- Figur 6:: eine Teilansicht eines Behälters des Isolators der Figur 3,
- Figur 7:: eine Prinzip-Darstellung für die Umsetzung des NFT-Verfahrens in einem Karussell-Isolator,
- Figur 8:: einen Behälter des Isolators der Figuren 1 und 2,
- Figur 9:: eine Draufsicht auf einen Isolator der Figuren 1 und 2 beim erfindungsgemäßen Umsetzen.

Die erfindungsgemäße Wechselstation bzw. der erfindungsgemäße Karussell-Isolator stellt eine miniaturisierte Haltungs-, Zucht- und Experimentplattform sowohl für die Tierforschung als auch für die Pflanzenforschung dar, die modular konfiguriert werden kann. Sie kann stark automatisiert sein. bzw. werden. Sie kann alle Funktionen zur Durchführung und Dokumentation von Tier- und Pflanzenversuchen bzw. deren Aufzucht und Vermehrung in einer ggf. zu 100% kontrollierten und keimfreien Umgebung (Reinraum) über den gesamten Lebenszyklus integrieren, zum Beispiel auch bis hin zur Ernte und zum Verkauf.

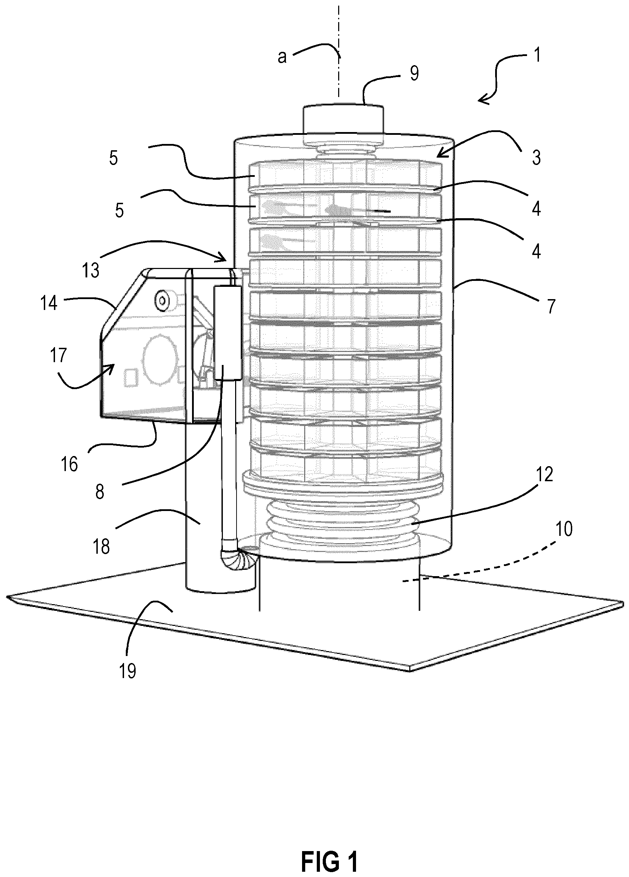

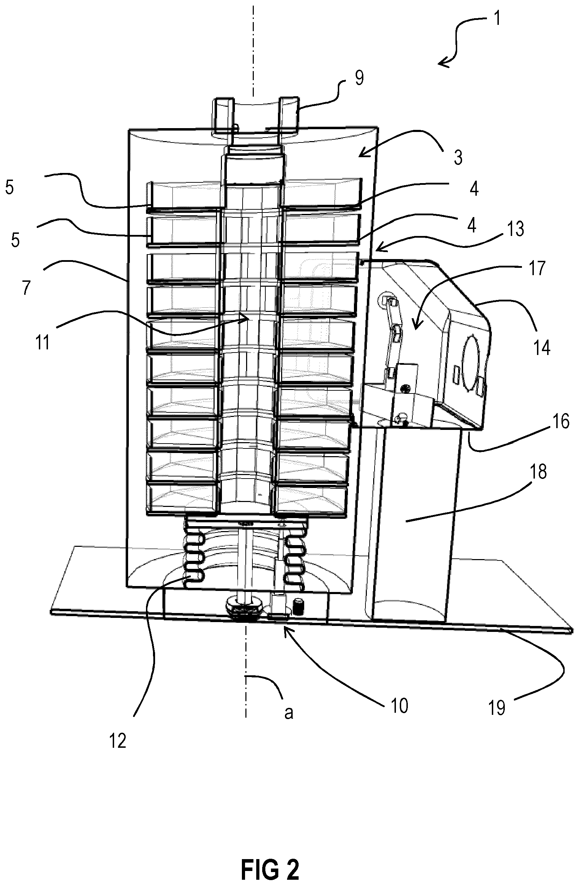

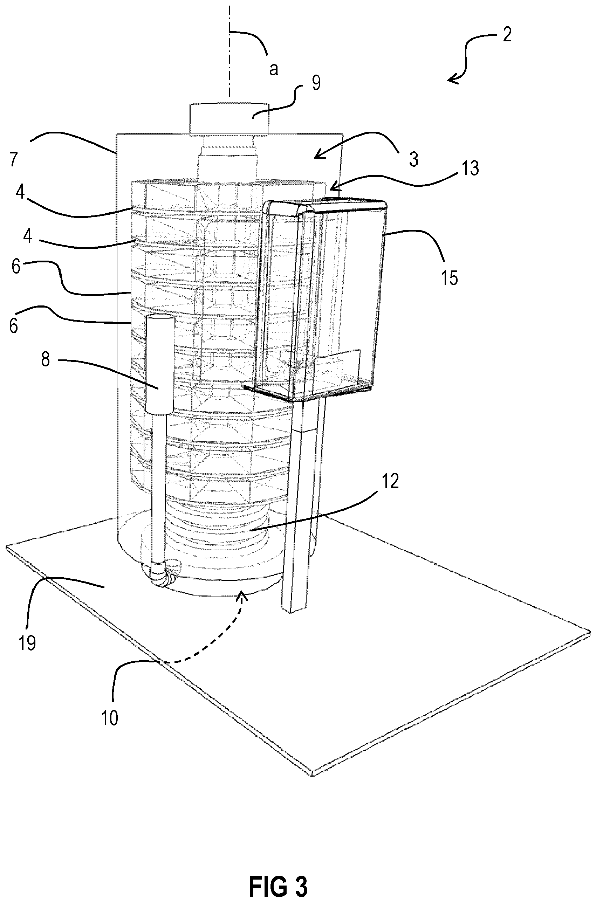

Der Aufbau der Wechselstationen 1, 2 der Figuren 1 bis 3 folgt weitgehend derjenigen der früheren Anmeldung WO 2018/007 596 desselben Anmelders. Die Offenbarung der WO 2018/007 596 wird daher auch zum Inhalt der vorliegenden Anmeldung gemacht. Wechselstationen 1 für die Tierforschung der Figuren 1, 2 und Wechselstationen 2 für die Pflanzenforschung der Figur 3 verfügen demnach übereinstimmend über ein dreh- und hebbares Karussellregal 3 als zylindrisches Regal mit verschiedenen Tellern 4, die (Teller-)Ebenen definieren, auf denen die tortenförmigen Tierkäfige 5 bzw. Pflanzencontainer 6 (im Folgenden gemeinsam als Behälter 5, 6 bezeichnet) untergebracht sind. Die Teller 4 lassen sich aufeinander stecken und bilden zusammen im Zentrum eine hohle Säule 11 (Fig. 2). Die Säule 11 wird für das Abführen der Luft, für Rohre eines Wasserkreislaufs sowie für Elektrokabel verwendet. In der Säule 11 verläuft eine Hochachse a als Dreh- und Hubachse des Karussellregals 3. Zur ihrer oberseitigen Stabilisierung stützt sich die Säule 11 mit einer teleskopierbaren Verlängerung an der Hülle 7 ab.

Ein Filter 8 und ein Gebläse 9 mit integrierten zweiteiligen Edelstahl-Kerzenfiltern und nicht dargestellte Lichttechnik sorgen für die Aufrechterhaltung eines definierbaren Klimas und Hygienestatus in einer Hülle 7 des Karussellregals 3. Ist der Innenraum der Hülle 7 hermetisch abschließbar, bietet er einen Reinraum. Zuluft gelangt über zwei seitlich neben der Hülle 7 angebrachte Schwebstoff- bzw. HEPA-Filter 8 in die Hülle 7. Die Abluft saugt das ebenfalls mit einem HEPA-Filter ausgestatten Gebläse 9 auf einer Oberseite der Hülle 7 bzw. des Karussellregals 3 ab. Die Wechselstationen 1, 2 können sowohl im Überdruck als auch im Unterdruck gefahren werden. Gebläse für Zu- und Abluft dienen dann der Erhaltung des gewünschten Drucks insbesondere innerhalb eines Isolators. Details der Wirkung der Gebläse auf die Behälter 5, 6 und das Zusammenwirken mit ihnen sind auch der WO 2018/007 596 zu entnehmen, die auch zum Gegenstand der vorliegenden Anmeldung gemacht werden.

Das Karussellregal 3 dreht sich mit einem Rotor, der auf einem Stator gelagert ist. Das Karussellregal 3 und der Rotor sind von der Hülle 7 umgeben, die im unteren Teil ein Balg 12 ist und auf einer die Wechselstation 1, 2 tragenden Grundplatte 19 ruht. Details des vom Reinraum innerhalb der Hülle 7 isolierten Antriebs 10, der für die automatisierbaren Dreh- und Hubbewegungen des Karussellregals 3 sorgt, sind ebenfalls der WO 2018/007 596 zu entnehmen, die ebenso zum Gegenstand der vorliegenden Anmeldung gemacht werden.

Die an sich bekannte Technologie klassischer Tierfolienisolatoren mit Überdruck findet auch bei den Wechselstationen 1, 2 Anwendung. Üblich sind zwei Varianten für die Hülle 7, nämlich entweder eine geschweißte PVC-Blase ("softwalled"), die auf einem Gestell montiert ist, oder eine feste Hülle ("hardwalled") z. B. aus Acryl, die sich aus verschiedenen Elementen zusammensetzen lässt (vgl. WO 2018/007 596, Fig. 1, 2 samt zugehöriger Beschreibung). Als Sterilisationsverfahren kommen sowohl für "hard-" als auch für "soft-walled"- Isolatoren das Versprühen von 2-prozentiger Per-Essigsäure-Lösung oder das Begasen mit Wasserstoffperoxid-Gas in Frage.

Die Hülle 7 der Wechselstation 1 (Fig. 1, 2) verfügt über eine Schnittstelle 13 zum Anschluss eines Handschuhkastens 14, dessen Innenraum ggf. zum Reinraum der Wechselstation 1 gehört. Er bietet eine ebene Arbeitsplatte 16 zur Manipulation der Käfige 5. (Nicht dargestellte) Neoprenhandschuhe ermöglichen ein einfaches Arbeiten im Innenraum des Handschuhkastens 14. Die Handschuhe sind im Falle von Beschädigungen austauschbar und müssen regelmäßig kontrolliert werden, da sie die größte Schwachstelle darstellen. Deshalb sollten im Betrieb frisch autoklavierte Baumwollhandschuhe als Überziehhandschuhe verfügbar sein, um eine Beschädigung der Neoprenhandschuhe möglichst zu vermeiden.

Das Versorgen mit und Entsorgen von Material erfolgt über z.B. (nicht dargestellte) DTPE^{®}-Ports oder Transferschleusen. Sie können für einen keimfreien Materialtransfer auf den beiden Schmalseiten 17 des Handschuhkastens 14 angebracht sein. Ein Abfallbehälter 18 kann auf der Unterseite der Arbeitsplatte 16 angebracht sein. Bzgl. des Handschuhkastens 14, der Handschuhe, der DTPE^{®}-Ports, der Transferschleusen und des Abfallbehälters 18 bzw. alternativer Sicherheitsbehälter wird ergänzend auf die WO 2018/007 596 verwiesen, deren diesbezügliche Offenbarung auch zum Gegenstand der vorliegenden Anmeldung gemacht wird.

Auch die Hülle 7 einer Wechselstation für die Pflanzenforschung kann über eine Schnittstelle 13 zum Anschluss eines Handschuhkastens 14 verfügen, um die Container 6 ggf. unter Reinraumbedingungen zu bearbeiten. Die Hülle 7 der Wechselstation 2 (Fig. 3) hat ebenfalls eine Schnittstelle 13, an der jedoch abweichend davon eine Ausgabeeinrichtung 15 für eine z. B. manuelle Entnahme angebracht ist. Damit kann die Wechselstation 2 eine völlig neue Produktions-, Ernte und Verkaufsplattform für ein "Vertikal Farming" darstellen, die alle Funktionen zur Aufzucht von Pflanzen von der Keimung der Samen bis zur Ernte integrieren kann.

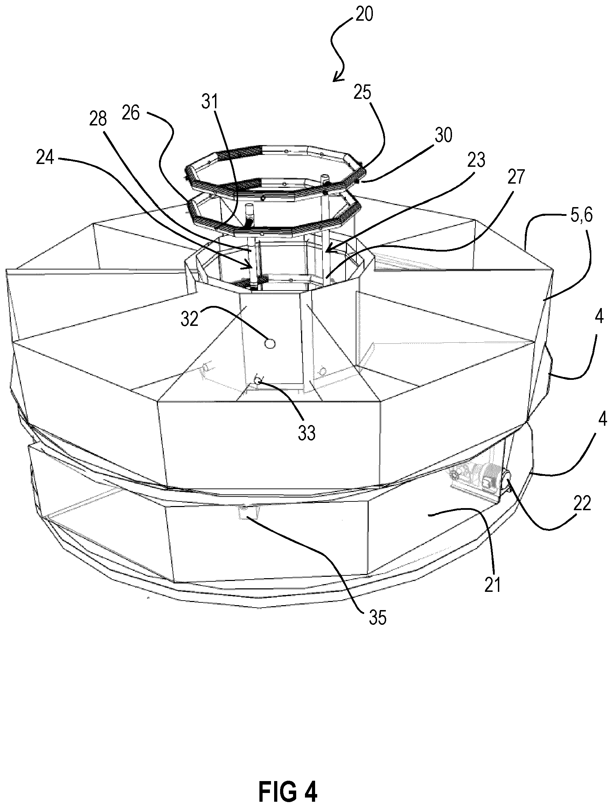

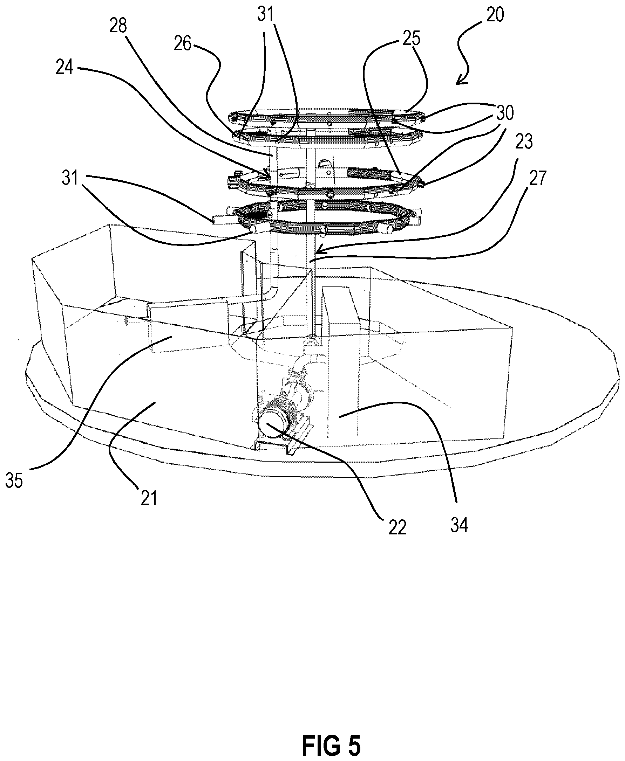

Sowohl für Wechselstationen 1 für die Tierforschung der Figuren 1, 2 als auch für Wechselstationen 2 für die Pflanzenforschung ist erfindungsgemäß mit einer zentralen Wasserversorgungseinrichtung 20 gemäß Figuren 4, 5 ausgestattet. Figur 4 zeigt einen unteren Teller 4 eines Karussellregals 3 mit einigen Bestandteilen der Wasserversorgungseinrichtung 20, die aber teilweise von einem ersten, darüber angeordneten Teller 4 mit Behältern 5, 6 verdeckt sind. Figur 5 dagegen gibt nur den untersten Teller 4 wieder. Die Wasserversorgungseinrichtung 20 umfasst demnach einen Wasservorratstank 21, der in der Horizontalen den Raum mehrerer Behälter 5, 6 einnimmt, dessen Höhe aber der Höhe der Behälter 5, 6 entspricht. Er passt damit auf einen Teller 4. Er ist fluidisch mit einer Wasserpumpe 22 gekoppelt. Deren Leistung ist darauf ausgelegt, Wasser bis auf zum obersten Teller 4 des Karussellregals 3 zu pumpen.

An die Wasserpumpe 22 schließt sich druckseitig ein rohrförmiger und in Strömungsrichtung des Wassers vertikal aufsteigender Leitungsabschnitt, nämlich eine Steigleitung 27 als Bestandteil des Vorlaufs 23 an, der Wasser vom Wasservorratstank 21 zu jedem Behälter 5, 6 transportiert. Ein davon getrennter rohrförmiger Leitungsabschnitt bzw. ein Fallrohr 28 als Bestandteil des Rücklaufs 24 leitet Wasser von jedem Behälter 5, 6 zum Wasservorratstank 21 zurück. Es verläuft weitgehend parallel zum Steigrohr 27, in Strömungsrichtung betrachtet also vertikal abwärts.

Die Verteilung des Wassers des Vorlaufs 23 auf die Behälter 5, 6 eines Tellers 4 übernimmt eine ringförmige Verteilerleitung 25. Sie ist damit Bestandteil des Vorlaufs 23. Die Entsorgung des Wassers der Behälter 5, 6 derselben Tellers 4 leistet eine parallel dazu und darunter angeordnete ringförmige Verteilerleitung 26 als Bestandteil des Rücklaufs 24. Jedem Teller 4, der Behälter 5, 6 trägt, ist also jeweils ein konzentrisch angeordnetes, ringförmiges Paar aus einer Verteilerleitung 25 und einer Verteilerleitung 26 zugeordnet.

Die Verteilerleitung 25, 26 tragen fluidleitende Kopplungsstellen 30, 31 zu den Behältern 5, 6, die ihrerseits über entsprechende und damit koppelbare Gegenkopplungsstellen 32, 33 verfügen. Die Verteilerleitung 25 trägt eine erste Kopplungsstelle 30, die als Bestandteil des Vorlaufs 23 für einen Wasserzufluss zum Behälter 5, 6 sorgt. Sie wirkt mit der oberen Gegenkopplungsstelle 32 am Behälter 5, 6 sowohl fluidisch als auch mechanisch zusammen. In entsprechender Weise trägt die darunterliegende Verteilerleitung 26 eine zweite Kopplungsstelle 31, die als Bestandteil des Rücklaufs 24 für einen Wasserabfluss vom Behälter 5, 6 sorgt und mit der unteren Gegenkopplungsstelle 33 am Behälter 5, 6 zusammenwirkt.

Die Behälter 5, 6 lassen sich radial aus dem Karussellregal 3 entnehmen und auf der Arbeitsplatte 16 servieren. Damit lösen sich die Gegenkopplungsstellen 32, 33 von den Kopplungsstellen 30, 31 und stellen insoweit nicht nur eine fluidische, sondern auch eine mechanische Schnittstelle dar, die beim Zurückstellen der Behälter 5, 6 in das Karussellregal 3 wieder genau aufeinanderpassen muss und eine zuverlässige Positionierung der Behälter 5, 6 auf den Tellern 4 und damit ihren bestimmungsgemäßen Anschluss an die Wasser- und Luftversorgung der Wechselstation 1, 2 sicherstellt.

Alle Bestandteile der Wasserversorgungseinrichtung 20, die den Vorlauf 23 und den Rücklauf 24 betreffen, sind in der Säule 11 (Fig. 2) untergebracht. Auf dem untersten Teller 4 dagegen sind noch eine Desinfektionseinheit 34 mit einer UV-Bestrahlungsanlage zur Keimvernichtung untergebracht. Sie ist druckseitig an der Wasserpumpe 22 angeflanscht und geht stromabwärts in die Steigleitung 27 des Vorlaufs 23 über. Der Rücklauf 24 endet nicht unmittelbar im Wasservorratstank 21, sondern in einem Abwasserfilter 35. Damit kann ein geschlossener Wasserkreislauf in den Wechselstationen 1, 2 hergestellt und aufrechterhalten werden.

Der unterste Teller 4 des Karussellregals 3 kann als Versorgungsebene dienen. Bei automatischer Wasserversorgung ist hier der Wasservorratstank 21 für die Versorgung der Tiere bzw. der Pflanzen gelagert. Um den Wasserdruck für die Versorgung der Behälter 5, 6 auszunützen, kann der Wassertank 21 je nach verfügbarer Deckenhöhe der Wechselstation 1, 2 auch auf einem oberen Teller 4 gelagert sein. Der Wasserverbrauch kann durch das zirkulierende Verfahren in einem geschlossenen Wasserkreislauf minimiert werden. Der unterste Teller 4 als Versorgungsebene dreht sich mit dem Rotor des Antriebs 10 (Fig. 1 bis 3) mit. Die Versorgung der Wasserpumpe 22 und der Desinfektionseinheit bzw. UV-Bestrahlungsanlage 34 mit Strom ist von oben von unten durch die Säule 11 hindurch vorgesehen. Durch das UV-Licht der Desinfektionseinheit 34 kann das Wasser keimfrei in den Versorgungsteil des Kreislaufs für die Tiere bzw. Pflanzen gebracht werden. Das Wasser des Rücklaufs 24 wird vor dem Eintreten in den Wasservorratstank 21 z. B. durch einen Aktivkohlefilter im Abwasserfilter 35 gereinigt.

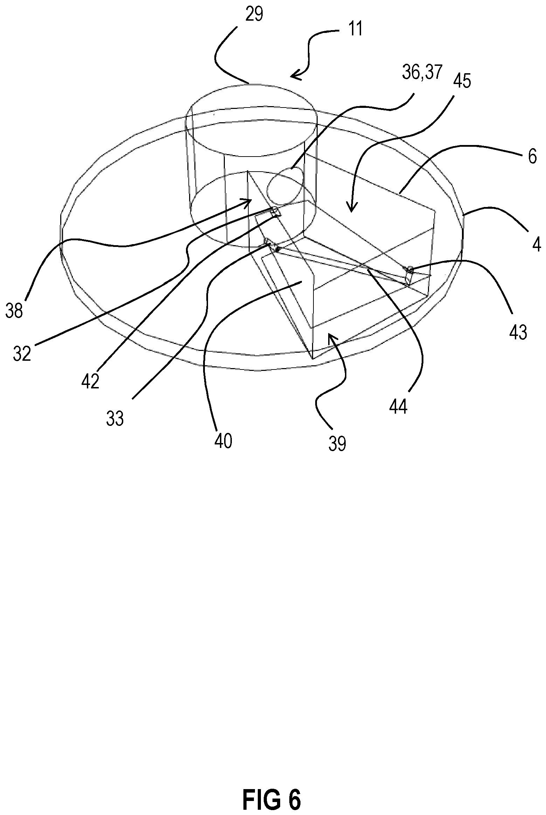

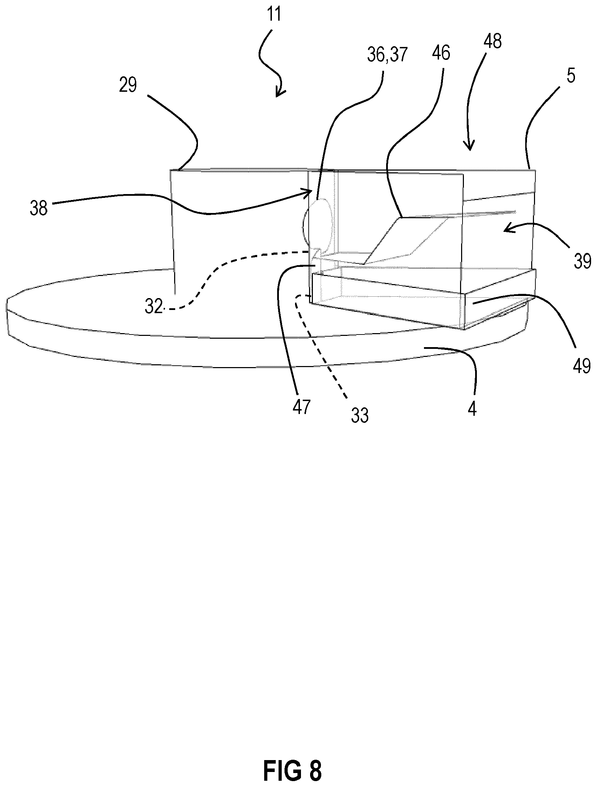

Anders als für das Wasser lässt sich innerhalb der Hülle 7 ein unidirektionaler Luftstrom aufrechterhalten, der sich aus den zwei seitlich neben der Hülle 7 angebrachten Filtern 8 für Zuluft und dem filtergestützten Gebläse 9 für Abluft speist (Fig. 1 bis 3). Beide Wechselstationen 1, 2 enthalten in der Säule 11 einen horizontal gerichteten Luftdurchlass 36 (Fig. 6, 8), der jedem Stellplatz eines Behälters 5, 6 auf den Tellern 4 zugeordnet ist. Jeder Behälter 5, 6, verfügt über einen entsprechenden Luftauslass 37 (Fig. 6, 8), der sich beim bestimmungsgemäßen Abstellen des Behälters 5, 6 auf einem Teller 4 mit dem Luftdurchlass 36 in der Säule 11 zur Deckung bringen lässt. Die Luft wird durch den Luftdurchlass 36 und durch den Luftauslass 37 aus jedem Behälter 5, 6 und über einen (nicht dargestellten) Luftspalt jedes Behälters 5, 6 aus dem Innenraum der Hülle 7 an- und nach oben abgezogen. Den Luftzug erzeugt das Gebläse 9 am oberen Ende der Säule 11. Dadurch sind sowohl Pflanzen als auch Tiere gut belüftet.

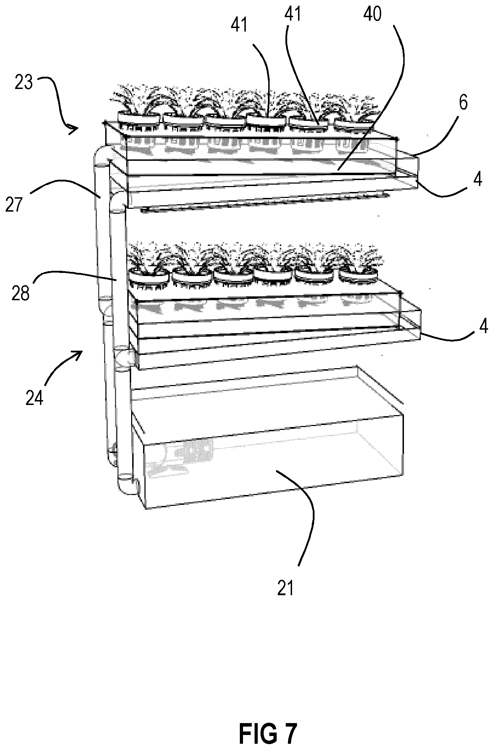

Soweit die Übereinstimmungen der Wechselstationen 1, 2, die unabhängig davon sind, ob die Wechselstationen 1, 2 für die Tierforschung oder für die Pflanzenforschung verwendet werden. Die unterschiedliche Verwendung kann ausschließlich auf den Käfigen 5 bzw. Container 6 beruhen bzw. auch nur in deren Ausstattung, die sich durch Einsätze der Behälter 5, 6 verändern lässt, wie die Figuren 6, 7 zeigen:
Figur 6 gibt - sehr vereinfacht - einen einzelnen Teller 4 wieder, der zentral einen Zylinderabschnitt 29 der Säule 11 umfasst. Darin ist - der Einfachheit halber nur ein - Luftdurchlass 36 zu erkennen. Die Bestandteile des Vorlaufs 23 und des Rücklaufs 24 (s. Fig. 4, 5) sind der besseren Übersicht wegen weggelassen.

Auf dem Teller 4 steht ein schematisierter Container 6. Er dockt mit seinem Luftauslass 37, der sich einer der Säule 11 zugewandten Schmalseite 38 des Containers 6 befindet, an den Luftdurchlass 36 der Säule 11 an. Unterhalb des Luftauslasses 37 verläuft eine zur gegenüberliegenden Schmalseite 39 hin abwärts geneigte Platte 40 als schiefe Ebene. Auf der Platte 40 lässt sich eine Vielzahl an Pflanzencontainern 41 (Fig. 7) abstellen. Auf einer Oberseite der Platte 40 mündet die obere Gegenkopplungsstelle 32 des Containers 6, die an der ersten Kopplungsstelle 30 des Vorlaufs 23 (s. Fig. 4, 5) angeschlossen ist. Darauf sitzt ein Verteilerkopf 42, der das Wasser des Vorlaufs 23 auf der Oberseite der Platte 40 verteilt.

An der tiefsten Stelle der Platte 40 sammelt sich unbenutztes Wasser. Dort ist an der Platte 40 ein Ablauf 43 ausgebildet, an den ein Rücklaufrohr 44 anschließt. Das Rücklaufrohr 44 koppelt an der unteren Gegenkopplungsstelle 33 des Containers 6 an, um das unbenutzte Wasser über die untere Kopplungsstelle 31 in den Rücklauf 24 (s. Fig. 4, 5) abzuleiten.

Die geneigte Platte 40, der Verteilerkopf 42, der Ablauf 43 und das Rücklaufrohr 44 sind als modularer oder als zusammenhängender Einsatz 45 ausgebildet, der einen beliebigen Behälter als Container 6 für die Pflanzenforschung ausstattet. Er lässt sich nicht nur zum erstmaligen Ausstatten des Containers 6, sondern auch zu Reinigungszwecken entnehmen.

Figur 7 veranschaulicht anhand eines stark vereinfachten Ausschnitts aus einer Wechselstation 2, wie sich das Prinzip des NFT- (Nutrient-Film-Technik) -Verfahrens erfindungsgemäß umsetzen lässt. Beispielhaft sind nur zwei übereinander angeordnete Container 6 auf Tellern 4 und mit dem Steigrohr 27 als Teil des Vorlaufs 23 und dem Fallrohr 28 als Teil des Rücklaufs 24 zum Wasservorratstank 21 dargestellt. Der Vorlauf 23 versorgt den Container 6 mit frischem Wasser bzw. sauerstoffreicher Nährlösung. Die Verteilerköpfe 42 benetzen die Oberfläche der Platte 40 gleichmäßig und versorgen damit die Wurzeln der Pflanzen in den Pflanzcontainern 41. Unbenutzte Flüssigkeit gelangt zurück in den Wasservorratstank 21, wo es ggf. nach einer Zwischenbehandlung wieder als Frischwasser bzw. Nährlösung zur Verfügung steht.

Alternativ zum dargestellten NFT-Verfahren kann die Versorgung der Samen, Keimlinge und Pflanzen mit Wasser und Nährstoffen auch mit Hilfe des Ebbe-/Flutverfahrens erfolgen (nicht dargestellt). Im Falle des Ebbe-/Flutverfahrens werden die Wurzeln nicht permanent durch das zirkulierende Wasser wie beim NFT-Verfahren benetzt, sondern schubweise für eine bestimmte Zeit. In diesem Fall umfasst der Behältereinsatz keine schiefe Ebene, sondern eine von dem Steigrohr 27 versorgte Zuflusskammer mit einer Abflussöffnung, die in eine darunterliegende Abflusskammer führt, die in das Fallrohr 28 entwässert. Ein Containerhalter positioniert die Pflanzencontainer 41 in der Zuflusskammer.

Die Flüssigkeit kann also bei geringstem Wasserverbrauch in einem geschlossenen Wasserkreislauf geführt werden. Optional ist vorgesehen, die Container 6 einzelner Ebenen bzw. Teller 4 mit unterschiedlichen Nährlösungen zu versorgen und deren elektrische Leitfähigkeit permanent zu kontrollieren, um den Pflanzen auf der jeweiligen Ebenen bzw. Tellern 4 eine optimale Osmose zu erlauben.

Die erfindungsgemäße Wechselstation 2 kann damit eine völlig neue Produktions-, Ernte und sogar Verkaufsplattform für das "Vertikal Farming" darstellen: Sie integriert alle Funktionen zur Aufzucht von Pflanzen von der Keimung der Samen bis zur Ernte. Durch eine Automatisierung der Wechselstation 2 mit Hilfe des Dreh- und Hubmechanismus des Antriebs 10 (Fig. 3) in Verbindung mit einem vertikal laufenden robotischen Pickmechanismus in der Ausgabeeinrichtung 15 können vollständige Container 6 serviert werden, aus denen ein Benutzer dann einzelne Pflanzen entnehmen kann. Über eine Fotozelle kann beim Zurückstellen des Containers 6 kontrolliert werden, ob die Anzahl der angeforderten Pflanzen den entnommenen Pflanzen entspricht. Alternativ kann ein ausgewählter Pflanzencontainer 41 auf Anforderung eines Kunden hin robotisch aus einem Container 6 entnommen und zur händischen Entnahme in der Ausgabeeinrichtung 15 angeboten werden.

Optional kann ein Roboter oder Cobot ein vor der Ausgabeeinrichtung 15 angeordnetes Ernte- bzw. Verkaufsregal vorab bestücken, indem einzelne Pflanzencontainer 41 mit Hilfe des Roboters bzw. Cobots oder eines erweiterten Pickmechanismus automatisch dem Karussellregal 3 entnommen und im Verkaufsregal platziert werden. In diesem Fall entnimmt der Kunde die Pflanzencontainer 41 dem Verkaufsregal und registriert sie, wie heute bereits in Supermärkten üblich. Damit kann Personal für die Bonnierung und Ernte weitgehend eingespart werden.

Figur 8 zeigt stark schematisiert einen Käfig 5 für die Tierforschung, der in seiner bestimmungsgemäßen Position auf einem Teller 4 abgestellt ist. Mit seiner Schmalseite 38 liegt er an dem Zylinderabschnitt 29 der Säule 11 an, um sowohl an die Luftversorgung über den Luftdurchlass 36 und den Luftauslass 37 als auch an die (nicht dargestellte) Wasserversorgung angeschlossen zu sein.

Der Käfig 5 enthält ein über wiegend waagrecht verlaufendes, doppelt gekröpftes Gitter 46, das von der inneren Schmalseite 38 des Käfigs 5 zur gegenüberliegenden äußeren Schmalseite 39 hin ansteigt. Seine Breite entspricht derjenigen des Käfigs 5. Das Gitter 46 sitzt an der inneren Schmalseite 38 unterhalb des Luftauslasses 37 des Käfigs 5, so dass Tiere den Käfig 5 nicht durch den Luftauslass 37 hindurch verlassen können.

Die obere Gegenkopplungsstelle 32 (s. auch Fig. 4) des Käfigs 5 ist über ein aufsteckbares U-förmiges Rohrstück 47 mit der unteren Gegenkopplungsstelle 33 (s. auch Fig. 4) verbunden. An dem U-förmigen Rohrstück 47 befindet sich ein an sich bekanntes Trinkventil, über das Tiere Trinkwasser zapfen können. Dazu ist das Trinkventil an dem U-förmigen Rohrstück 47 so positioniert, dass es sich unterhalb des Gitters 46 befindet.

Auf dem Boden des Käfigs 5 steht ein schalenförmiger Einstreueinsatz 49. Er ist separat entnehmbar, um Einstreumaterial zur Aufnahme von Ausscheidungen der Tiere einfach austauschen zu können.

Das Gitter 46, das U-Stück 47 mit dem Trinkventil daran und der Einstreueinsatz 49 stellen zusammen einen zerlegbaren oder einstückigen Einsatz 48 dar, der zur Ausstattung eines zunächst beliebigen Behälters zu einem Käfig 5 für die Tierforschung dient. Der Einsatz 48 kann z.B. zu Reinigungszwecken separat entnommen werden. Er kann je nach Einsatz- oder Forschungszweck in der Tierforschung auch abweichend aufgebaut sein, beispielsweise ein abweichend gestaltetes Gitter 46, evtl. mit einer Ausbuchtung für Futter, umfassen.

Das Konzept der Käfige 5 mit ggf. unterschiedlichen Einsätzen 48 erlaubt es, jeweils vorbereitete Einsätze 48 mit Einstreu und Käfiggitter 46 mit Nahrung jeweils gestapelt einzuschleusen und auf den Tellern 4 zu lagern. Die vorbereitete Einsätze 48 können dem Bediener oder einem Cobot vom Dreh-Hubmechanismus des Antriebs 10 zur weiteren Verarbeitung serviert werden.

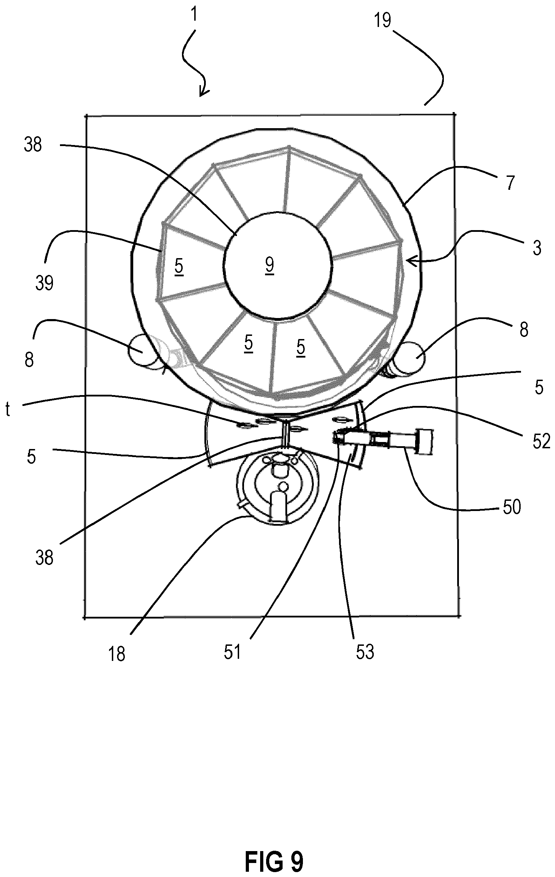

Figur 9 bietet eine vereinfachte Draufsicht auf eine Wechselstation 1. Auf der Grundplatte 19 steht das Karussellregal 3, das die zylindrische Hülle 7 umgibt, auf deren Oberseite das Gebläse 9 für die Abluft installiert ist. In der Draufsicht der Figur 9 lassen sich die ringförmig um die verdeckte Säule 11 (Fig. 2) angeordneten, weitgehend sektorförmigen Käfige 5 erkennen, deren kürzeren Schmalseiten 38 an der Säule 11 anliegen (und deren Anordnung derjenigen der Container 6 in einer Wechselstation 2 entspricht).

Beidseits der Hülle 7 sind die Filter 8 für Zuluft angeordnet. Dazwischen liegt am Umfang der Hülle 7 ein Arbeitsbereich zur Manipulation der Käfige 5, der der Einfachheit halber ohne Arbeitsplatte und ohne Handschuhkasten dargestellt ist. Er enthält zwei Käfige 5 mit Tieren t darin, den zylindrischen Abfallbehälter 18 unterhalb der Arbeitsebene und einen Roboterarm 50 eines Roboters oder eines Cobot, an dem eine Kamera 51 und ein Greifer 52 angebracht sind. Der Greifer 52 hat ein leicht gebogenes Blechschild, alternativ einen Fächer oder eine Blasvorrichtung als Tierführung 53 ergriffen.

Die beiden Käfige 5 im Arbeitsbereich stehen mit ihren Schmalseiten 38 einander zugewandt und liegen direkt aneinander an. Die Schmalseiten 38 enthalten Schiebetüren, die Öffnungen in den Seitenwänden 38 öffnen und verschließen können.

Nach wissenschaftlichen Erkenntnissen gelingt das schonendste Verfahren des Umsetzens von Tieren t mit Handling-Röhrchen. Die erfindungsgemäße Wechselstation 1 erübrigt auch noch die Handling-Röhrchen: Der Cobot ordnet die Käfige 5 mit den Einsätzen 48 jeweils mit ihrer Schmalseite 38 so an, dass ein Übergang entstehen kann. Dann öffnet der Cobot die ggf. mit Luftfiltern versehenen Schiebetüren oder Fallgatter beider sich gegenüberstehenden Käfige 5 und motiviert die Tiere t zum Käfigwechsel. Dazu kann er die Tierführung 53 unterstützend einsetzten. Neugierige Tiere t werden bereits von allein wechseln.

Nach dem Wechsel der Tiere t in den neuen Käfig 5 schließt der Cobot das Fallgatter und entnimmt den Einsatz 48 mit der alten Einstreu und lässt diesen in den geöffneten Abfallbehälter 18 an der Unterseite der Arbeitsfläche an einer Schiene nach unten gleiten. Dort stapeln sich die verbrauchten Einsätze 48.

Die Käfige 5 lassen sich variieren, nämlich zum Beispiel mit einem - auch manuell zu betätigenden - verschiebbaren Verschluss des Durchbruchs als horizontal verfahrbare Schiebetüren oder vertikal bewegliche Fallgatter, durch ein luftdurchgängiges Gitter oder Filter im oder als Verschluss, so dass der Durchbruch zugleich als Luftauslass 37 (Fig. 6, 8) dienen kann und/oder durch (nicht dargestellte) schildförmige Leitplatten, die in den beiden Ecken des Käfigs 5 beidseits des Durchbruchs/Luftauslasses 37 einsetzbar sind und die die Behälterecken abschirmen.

Da es sich bei den vorhergehenden, detailliert beschriebenen Wechselstationen 1, 2 um Ausführungsbeispiele handelt, können sie in üblicher Weise vom Fachmann in einem weiten Umfang modifiziert werden, ohne den Bereich der Erfindung wie in den beiliegenden Ansprüchen definiert zu verlassen. Insbesondere können auch die konkreten Ausgestaltungen der Behälter 5, 6 oder ihrer Einsätze 45, 48 in anderer Form als in der hier beschriebenen erfolgen. Ebenso kann der Handschuhkasten 14, die Ausgabeeinrichtung 15 oder der Abfallbehälter 18 in einer anderen Form ausgestaltet werden, wenn dies aus Platzgründen bzw. gestalterischen Gründen notwendig ist. Außerdem schließt die Verwendung der unbestimmten Artikel "ein" bzw. "eine" nicht aus, dass die betreffenden Merkmale auch mehrmals oder mehrfach vorhanden sein können.

### Bezugszeichenliste

- 1: Wechselstation für die Tierforschung
- 2: Wechselstation für die Pflanzenforschung
- 3: (Karussell-)Regal
- 4: Teller
- 5: (Tier-)Käfig, Behälter
- 6: (Pflanzen-)Container, Behälter
- 7: Hülle
- 8: Filter
- 9: Gebläse
- 10: Antrieb
- 11: Säule
- 12: Balg
- 13: Schnittstelle
- 14: Handschuhkasten
- 15: Ausgabeeinrichtung
- 16: Arbeitsplatte
- 17: Schmalseite
- 18: Abfallbehälter
- 19: Grundplatte
- 20: Wasserversorgungseinrichtung
- 21: Wasservorratstank
- 22: Wasserpumpe
- 23: Vorlauf
- 24: Rücklauf
- 25: ringförmige Leitung; Verteilerleitung
- 26: ringförmige Leitung; Sammelleitung
- 27: Steigrohr
- 28: Fallrohr
- 29: Zylinderabschnitt
- 30: Kopplungsstelle
- 31: Kopplungsstelle
- 32: obere Gegenkopplungsstelle
- 33: untere Gegenkopplungsstelle
- 34: Desinfektionseinheit
- 35: Abwasserfilter
- 36: Luftdurchlass
- 37: Durchbruch; Luftauslass
- 38: Schmalseite
- 39: Schmalseite
- 40: schiefe Ebene; Platte
- 41: Pflanzencontainer
- 42: Verteilerkopf
- 43: Ablauf
- 44: Rücklaufrohr
- 45: Einsatz
- 46: Gitter
- 47: U-förmigen Leitung; Rohrstück
- 48: Einsatz
- 49: Einstreueinsatz
- 50: Roboterarm
- 51: Kamera
- 52: Greifer
- 53: Tierführung

## Patentansprüche

1. Als Reinraumeinrichtung ausgebildete Zuchteinrichtung bzw. Wechselstation zum Herstellen und zum Aufrechterhalten einer keimfreien Umgebung, für die Tier- oder Pflanzenforschung,
- mit einem drehbaren zylindrischen Regal (3) mit einer Hochachse (a) als Drehachse,
- mit scheibenförmigen Tellern (4) mit Stellplätzen zum Abstellen von Behältern (5; 6),
- mit einer Hubeinrichtung und mit einer Dreheinrichtung für die scheibenförmigen Teller (4),
- mit im Regal (3) ringförmig anzuordnenden und in radialer Richtung entnehmbaren im Wesentlichen sektorförmigen Behältern (5; 6),
- mit einer Hülle (7) zur sterilen Abschirmung des Regals (3) gegenüber der Umgebung und
- mit einer Schnittstelle (13) an der Hülle (7) zum Anschluss eines Handschuhkastens (14) oder eines Entnahmegestells (15),
**gekennzeichnet durch** eine Wasserversorgungseinrichtung (20) für die Behälter (5; 6), die vollständig innerhalb der Hülle der Wechselstation (1; 2) untergebracht ist und die eine Versorgung jedes Behälters (5; 6) ermöglicht.

2. Zuchteinrichtung nach Anspruch 1, **dadurch gekennzeichnet, dass** die Wasserversorgungseinrichtung (20) auf zumindest einem Teller des Regals angeordnet ist.

3. Zuchteinrichtung nach Anspruch 1 oder 2, **dadurch gekennzeichnet, dass** die Wasserversorgungseinrichtung (20) einen Wasservorratstank (21), eine Wasserpumpe (22), einen Vorlauf (23) vom Wasservorratstank (21) zu jedem Behälter (5; 6) und einen davon getrennten Rücklauf (24) von jedem Behälter (5; 6) zum Wasservorratstank (21) umfasst.

4. Zuchteinrichtung nach Anspruch 3, **dadurch gekennzeichnet, dass** jedem Teller (4) jeweils eine ringförmige Leitung (25) als Bestandteil des Vorlaufs (23) und eine ringförmige Leitung (26) als Bestandteil des Rücklaufs (24) zugeordnet ist.

5. Zuchteinrichtung nach einem der obigen Ansprüche, **gekennzeichnet durch** eine erste Kopplungsstelle (30) für einen Wasserzufluss zu einem der Behälter (5; 6) und einer zweiten Kopplungsstelle (31) für den Wasserabfluss von demselben Behälter (5; 6) und durch die Behälter (5; 6) mit jeweils zwei mit den Kopplungsstellen zusammenwirkenden Gegenkopplungsstellen (32; 33).

6. Zuchteinrichtung nach einem der obigen Ansprüche, **gekennzeichnet durch** einen geschlossenen Wasserkreislauf der Wasserversorgungseinrichtung (20).

7. Zuchteinrichtung nach einem der obigen Ansprüche, **gekennzeichnet durch** eine Reinigungseinrichtung (34; 35) für das Wasser der Wasserversorgungseinrichtung (20) und/oder durch eine Dosiereinrichtung für das Wasser der Wasserversorgungseinrichtung (20) und/oder durch eine Belüftungs- und/oder Klimatisierungseinrichtung.

8. Zuchteinrichtung nach Anspruch 5 oder nach einem der Ansprüche 6 oder 7, soweit auf Anspruch 5 bezogen, **gekennzeichnet durch** eine Anzahl der Behälter (6) mit einer schiefen Ebene (40) darin, mit Abstellplätzen für Pflanzen auf der schiefen Ebene (40), mit der Gegenkopplungsstelle (32) für den Wasserzufluss an einer Oberseite und mit der Gegenkopplungsstelle (33) für den Wasserabfluss an einer Unterseite der schiefen Ebene (40).

9. Zuchteinrichtung nach Anspruch 5 oder nach einem der Ansprüche 6 oder 7, soweit auf Anspruch 5 bezogen, **gekennzeichnet durch** eine Anzahl der Behälter (5) mit der Gegenkopplungsstelle (32) für den Wasserzufluss und mit der Gegenkopplungsstelle (33) für den Wasserabfluss, die mit einer U-förmigen Leitung (47) verbunden sind, die ein Trinkventil für Tiere aufweist.

10. Zuchteinrichtung nach Anspruch 8, **gekennzeichnet durch** die schiefe Ebene (40) und die oberseitige (32) und die unterseitige Gegenkopplungsstelle (33) als Teil eines austauschbaren Einsatzes (45) zum Erzeugen der Behälter (6) als Behältern (6) für die Pflanzenforschung.

11. Zuchteinrichtung nach Anspruch 9, **gekennzeichnet durch** die mit einer U-förmigen Leitung (47) verbundenen Gegenkopplungsstellen (32; 33) als Teil eines austauschbaren Einsatzes (48) zum Erzeugen der Behälter (5) als Behältern (5) für die Tierforschung.

12. Zuchteinrichtung nach einem der obigen Ansprüche, soweit nicht auf Anspruch 8 bezogen, **gekennzeichnet durch** einen Durchbruch (37) einer Seitenwand (38) der Behälter (5) zum Umsetzen von Tieren, wobei der Durchbruch (37) derart angeordnet ist, dass die Durchbrüche (37) zweier Behälter (5) bei einem benachbarten / Nebeneinander-Positionieren der Behälter (5) Seite an Seite miteinander zur Deckung kommen.

13. Zuchteinrichtung nach Anspruch 12, **gekennzeichnet durch** einen verschiebbaren Verschluss des Durchbruchs (37).

14. Zuchteinrichtung nach einem der Ansprüche 12 oder 13 mit den Behältern (5) mit einem Lufteinlass (37) an einer Schmalseite (38), **gekennzeichnet durch** den Lufteinlass (37) als Durchbruch (37) zum Umsetzten von Tieren.

15. Zuchteinrichtung nach einem der obigen Ansprüche, soweit nicht auf Ansprüche 9 oder 11 bis 14 bezogen, **gekennzeichnet durch** eine Ausgabeeinrichtung (15) für Pflanzen, insbesondere mit einem Industrieroboter zur Manipulation der Behälter (6) und ihres Inhalts innerhalb der Hülle (7) und einem außenseitigen Entnahmegestell zur benutzerseitigen Entnahme und mit einem Anschluss an die Schnittstelle (13) an der Hülle (7).

## Claims

1. Culture facility or changing station designed as a clean room facility for creating and maintaining a germ-free environment for animal or plant research,
- with a rotatable cylindrical shelf (3) with a vertical axis (a) as the axis of rotation,
- with disc-shaped plates (4) with spaces for placing containers (5; 6),
- with a lifting device and with a rotating device for the disc-shaped plates (4),
- with substantially sector-shaped containers (5; 6) to be arranged in an annular manner in the rack (3) and removable in a radial direction,
- with a cover (7) for sterile shielding of the shelf (3) from the environment and
- with an interface (13) on the cover (7) for connecting a glove box (14) or a removal rack (15),
**characterised by** a water supply device (20) for the containers (5; 6), which is accommodated completely within the shell of the exchange station (1; 2) and which enables each container (5; 6) to be supplied.

2. Culture facility according to claim 1, **characterised in that** the water supply device (20) is arranged on at least one plate of the rack.

3. Culture facility according to claim 1 or 2, **characterised in that** the water supply device (20) comprises a water supply tank (21), a water pump (22), a flow (23) from the water supply tank (21) to each container (5; 6) and a separate return flow (24) from each container (5; 6) to the water supply tank (21).

4. Culture facility according to claim 3, **characterised in that** each plate (4) is assigned an annular line (25) as a component of the feed line (23) and an annular line (26) as a component of the return line (24).

5. Culture facility according to one of the above claims, **characterised by** a first coupling point (30) for a water inflow to one of the containers (5; 6) and a second coupling point (31) for the water outflow from the same container (5; 6) and by the containers (5; 6) each having two counter-coupling points (32; 33) cooperating with the coupling points.

6. Culture facility according to one of the above claims, **characterised by** a closed water circuit of the water supply device (20).

7. Culture facility according to one of the preceding claims, **characterised by** a purification device (34; 35) for the water of the water supply device (20) and/or by a dosing device for the water of the water supply device (20) and/or by a ventilation and/or air-conditioning device.

8. Culture facility according to claim 5 or according to one of the claims 6 or 7, as far as related to claim 5, **characterised by** a number of the containers (6) with an inclined plane (40) therein, with storage places for plants on the inclined plane (40), with the counter-coupling point (32) for the water inflow on an upper side and with the counter-coupling point (33) for the water outflow on a lower side of the inclined plane (40).

9. Culture facility according to claim 5 or according to one of claims 6 or 7, as far as related to claim 5, **characterised by** a number of the containers (5) with the counter-coupling point (32) for the water inflow and with the counter-coupling point (33) for the water outflow, which are connected to a U-shaped conduit (47) comprising an animal drinking valve.

10. Culture facility according to claim 8, **characterised by** the inclined plane (40) and the top-side (32) and the bottom-side counter-coupling point (33) as part of an exchangeable insert (45) for creating the containers (6) as containers (6) for plant research.

11. Culture facility according to claim 9, **characterised by** the counter-coupling points (32; 33) connected to a U-shaped line (47) as part of an exchangeable insert (48) for creating the containers (5) as containers (5) for animal research.

12. Culture facility according to one of the above claims, insofar as not related to claim 8, **characterised by** an aperture (37) of a side wall (38) of the containers (5) for transferring animals, wherein the aperture (37) is arranged in such a way that the apertures (37) of two containers (5) coincide with each other when the containers (5) are positioned side by side.

13. Culture facility according to claim 12, **characterised by** a displaceable closure of the aperture (37).

14. Culture facility according to one of claims 12 or 13 with the containers (5) with an air inlet (37) on a narrow side (38), **characterised by** the air inlet (37) as an opening (37) for transferring animals.

15. Culture facility according to one of the above claims, insofar as not related to claims 9 or 11 to 14, **characterised by** a dispensing device (15) for plants, in particular with an industrial robot for manipulating the containers (6) and their contents inside the casing (7) and an external removal rack for user-side removal and with a connection to the interface (13) on the casing (7).

## Revendications

1. Dispositif d'élevage ou station de changement conçue comme un dispositf de salle blanche pour la création et le maintien d'un environnement stérile, pour la recherche animale ou végétale,
- avec un rayonnage cylindrique rotative (3) ayant un axe vertical (a) comme axe de rotation,
- avec des plats en forme de disque (4) avec des emplacements pour déposer des récipients (5; 6),
- avec un dispositif de levage et avec un dispositif de rotation pour les plats en forme de disque (4),
- avec des récipients (5; 6) essentiellement en forme de secteur à disposer en forme d'anneau dans le rayonnage (3) et à retirer dans la direction radiale,
- avec une enveloppe (7) pour la protection de manière stérile du rayonnage (3) par rapport à l'environnement et
- avec une interface (13) sur l'enveloppe (7) pour la connexion d'une boîte à gants (14) ou d'un support de prélèvement (15),
**caractérisé par** un dispositif d'alimentation en eau (20) pour les récipients (5; 6), entièrement logé à l'intérieur de l'enveloppe de la station de changement (1; 2) et permettant d'alimenter chaque récipient (5; 6).

2. Dispositif d'élevage selon la revendication 1, **caractérisé en ce que** le dispositif d'alimentation en eau (20) est disposé sur au moins un plat de Irayonnage.

3. Dispositif d'élevage selon la revendication 1 ou 2, **caractérisé en ce que** le dispositif d'alimentation en eau (20) comprend un réservoir de stockage d'eau (21), une pompe à eau (22), une conduite d'alimention (23) du réservoir de stockage d'eau (21) vers chaque récipient (5; 6) et une conduite de retour (24) séparé de celui-ci de chaque récipient (5; 6) vers le réservoir de stockage d'eau (21).

4. Dispositif d'élevage selon la revendication 3, **caractérisé en ce qu'**à chaque plat (4) est associé respectivement un conduit annulaire (25) faisant partie de la conduite d'alimentation (23) et un conduit annulaire (26) faisant partie d'une conduite de retour (24).

5. Dispositif d'élevage selon l'une des revendications précédentes, **caractérisé par** un premier point de couplage (30) pour une arrivée d'eau à l'un des récipients (5; 6) et un deuxième point de couplage (31) pour l'évacuation de l'eau du même récipient (5; 6) et par les récipients (5; 6) avec deux points de contre-couplage (32; 33) coopérant avec les points de couplage.

6. Dispositif d'élevage selon l'une des revendications ci-dessus, **caractérisé par** un circuit d'eau fermé du dispositif d'alimentation en eau (20).

7. Dispositif d'élevage selon l'une des revendications ci-dessus, **caractérisé par** un dispositif de purification (34; 35) de l'eau du dispositif d'alimentation en eau (20) et/ou par un dispositif de dosage de l'eau du dispositif d'alimentation en eau (20) et/ou par un dispositif d'aération et/ou de climatisation.

8. Dispositif d'élevage selon la revendication 5 ou selon l'une des revendications 6 ou 7, pour autant qu'il ne se réfère pas à revendication 5, **caractérisé par** un nombre des récipients (6) avec un plan incliné (40) à l'intérieur, avec des emplacements de stationnement pour les plantes sur le plan incliné (40), avec le point de contre-couplage (32) pour l'arrivée d'eau sur un côté supérieur et avec le point de contre-couplage (33) pour l'évacuation d'eau sur un côté inférieur du plan incliné (40).

9. Dispositif d'élevage selon la revendication 5 ou selon l'une des revendications 6 ou 7, pour autant qu'il ne se réfère pas à revendication 5, **caractérisée par** un nombre de récipients (5) avec le point de contre-couplage (32) pour l'arrivée de l'eau et avec le point de contre-couplage (33) pour l'évacuation de l'eau, qui sont reliés à un conduit en forme de U (47) qui comprend une valve d'abreuvement pour les animaux.

10. Dispositif d'élevage selon la revendication 8, **caractérisé par** le plan incliné (40) et le point de contre-couplage supérieur (32) et le point de contre-couplage inférieur (33) faisant partie d'un insert interchangeable (45) pour produire les récipients (6) en tant que récipients (6) pour la recherche sur les plantes.

11. Dispositif d'élevage selon la revendication 9, **caractérisé par** les points de contre-couplage (32; 33) reliés à un conduit en forme de U (47) faisant partie d'un insert interchangeable (48) pour produire les récipients (5) en tant que récipients (5) pour la recherche animale.

12. Dispositif d'élevage selon l'une quelconque des revendications ci-dessus, pour autant qu'il ne se réfère pas à revendication 8, **caractérisé par** une ouverture (37) d'une paroi latérale (38) des récipients (5) pour le transfert d'animaux, l'ouverture (37) étant disposée de telle sorte que les ouvertures (37) de deux récipients (5) se recouvrent entre elles lorsque les récipients (5) sont positionnés adjacent / côte à côte.

13. Dispositif d'élevage selon la revendication 12, **caractérisé par** une fermeture déplaçable de l'ouverture (37).

14. Dispositif d'élevage selon l'une des revendications 12 ou 13, comprenant les récipients (5) avec une entrée d'air (37) sur un petit côté (38), **caractérisé par** l'entrée d'air (37) sous forme d'un ouverture (37) pour le transfert d'animaux.

15. Dispositif d'élevage selon l'une des revendications ci-dessus, pour autant qu'il ne se réfère pas aux revendications 9 ou 11 à 14, **caractérisé par** un dispositif de distribution (15) pour des plantes, en particulier avec un robot industriel pour la manipulation des récipients (6) et de leur contenu à l'intérieur de l'enveloppe (7) et un support de prélèvement à côté extérieur pour le prélèvement à côté d'utilisateur et avec un raccordement à l'interface (13) sur l'enveloppe (7).
